# EUROPEAN PATENT SPECIFICATION

(11) **EP 3 840 826 B1**
(45) Date of publication and mention of the grant of the patent: **24.04.2024**
(21) Application number: 19851887.0
(22) Date of filing: 20.08.2019
(51) Int. Cl.: A61N 1/36, A61N 1/32, A61N 1/34, A61N 1/372

(54) **SYSTEM FOR ADJUSTING A THERAPEUTIC ELECTRICAL DOSE**
SYSTEM ZUR EINSTELLUNG EINER THERAPEUTISCHEN STROMDOSIS
SYSTÈME D'AJUSTEMENT D'UNE DOSE ÉLECTRIQUE THÉRAPEUTIQUE

(30) Priority: 20.08.2018 US 201862720071 P
(43) Date of publication of application: 30.06.2021
(73) Proprietor: Neuros Medical, Inc., Willoughby Hills, OH 44094 (US)
(72) Inventor: FANG, Zi-Ping, Willoughby Hills, OH 44094 (US); SYED SHAH, Nemath, Willoughby Hills, OH 44094 (US); IORIO, Matthew, J., Willoughby Hills, OH 44094 (US)
(74) Representative: V.O.
(86) International application number: PCT/US2019/047281
(87) International publication number: WO 2020/041323

(56) References cited:
- WO-A1-2018/067239
- US-A- 5 653 739
- US-A1- 2008 319 511
- US-A1- 2017 095 667
- US-A1- 2017 239 486
- US-A1- 2017 319 381
- US-A1- 2017 319 381
- US-A1- 2018 043 172
- US-A1- 2018 043 172
- US-B2- 8 676 331

## Description

### FIELD

The inventions described herein relate to the field of implantable neuromodulators, and in particular to the field of high-frequency nerve block.

### BACKGROUND

Implantable neuromodulators (e.g., implantable neurostimulators, implantable nerve block apparatus) are increasingly used to treat pain and other indications, in many cases by the direct application of electrical energy to one or more nerves, including nerve bundles. Such electrical modulation may be used to excite or inhibit nerves, or both. An implantable neuromodulator may be implanted on, around or adjacent to a patient's nerve or nerves for the delivery of electrical energy.

For example, electrical modulation may be applied to a nerve to treat the unwanted and/or uncoordinated generation of nerve impulses which may otherwise be a disabling factor in some medical conditions. Uncoordinated motor signals may produce spasticity in stroke, cerebral palsy, multiple sclerosis, and other conditions and may lead to pain, including pain resulting from amputation. The uncoordinated signals may result in the inability to make desired functional movements. Involuntary motor signals in conditions including tics, choreas, and so on, may produce unwanted movements. Unwanted sensory signals can cause pain.

Electrical modulation to treat a patient is generally sensitive to the amount, duration and intensity of the applied energy. For example, one non-limiting type of electrical therapy is applying high-frequency alternating current (HFAC) to nerves that have been shown to block nerve activity, e.g., in the treatment of pain. An appropriate dose (e.g., the amount of electrical energy applied to the patient for effective treatment) may be set so that it causes the desired effect, such as inhibition of nerve activity to reduce pain. On the other hand, an inappropriate dosing may lead to no effect or possibly to irritation of the nerve.

Unfortunately, determining proper dosing for a patient may be time-intensive, and complicated. Further, the optimal dosing to treat a patient may be highly variable between patients, and indeed, even over time in the same patient. Thus, it would be beneficial to provide a method and/or apparatus for simplifying and reliably adjusting patient dosing. Described herein are methods and apparatuses that may address these needs. Document US-B-8 676 331 discloses the most relevant prior art.

### SUMMARY OF THE DISCLOSURE

The invention is defined in claim 1. Any embodiment, which is in contradiction to the subject-matter of claim 1, is not part of the invention.

Described herein are computer-implemented methods and apparatuses (devices, systems, etc., including neuromodulators and systems including them) for setting the therapeutic dosing of a neuromodulator that is implanted into a patient and determined to treat pain. The present invention is characterized by the granted claims. A therapy dose may include a therapeutic dose duration, a dose frequency and a peak voltage (e.g., peak modulation voltage) and/or current. In some variation the dose may include a therapy ramp-up time to reach a peak modulation voltage and a sustained peak modulation time during which the voltage is sustained at the peak modulation voltage. The setting processes described herein may set the peak voltage to a voltage that is beyond the patient's nerve activation level and within a nerve blocking level. The dose parameters may also include the waveform parameters applied, e.g., pulsatile or repeating (e.g., sinusoidal, square wave, saw-tooth, biphasic, etc.), and the frequency of the applied waveform (e.g., the high-frequency component). Other dose parameters may include the initial (e.g., starting) voltage, which may be, e.g., zero, or may be a minimum patient-detectable modulation voltage that is determined as described herein. In some variations, the therapeutic dose includes at least two parts; an initial ramp-up portion in which the voltage increases from the initial voltage up to a peak modulation voltage, and a plateau portion, referred to as a sustained peak modulation time, during which the voltage is sustained at the peak modulation voltage. The duration of the ramp-up portion may be referred to as the ramp-up time. The duration of the second portion may be referred to as the plateau time. In some variations, these two portions may repeat and/or alternate.

The computer-implemented methods and apparatuses (e.g., systems) described herein may be configured to optimize the pain relief that may be achieved by an individual patient using an implanted neuromodulator (which may also be referred to herein as a neurostimulator, or implantable nerve block apparatus). In particular, these computer-implemented methods and apparatuses may be useful to set (e.g., optimize) the dosing parameters used by an implanted neuromodulator to provide relief from pain using high-frequency nerve block.

In the absence of the teachings described herein, it is difficult to determine an optimal dosage at which the applied voltage is increase to a peak and sustained for sufficiently long to achieve a therapeutic benefit. The inventors have found that although it is generally beneficial to apply as high a voltage as possible to the patient, particularly (but not exclusively) in applications in which neural modulation comprises high-frequency (e.g., greater than 1 kHz) modulation from an implanted neuromodulator to inhibit activity of a nerve or nerve bundle, there is a difficulty to define voltage threshold, above which further voltage amplitude may result in pain and/or discomfort for the patient. This threshold is not only variable between different patients, but may vary with respect to the individual patient.

In general the computer-implemented methods may be applied to, but are not limited to, the use with neuromodulation to provide a high-frequency block of a nerve or bundle of nerves. For example, the computer-implemented methods and apparatuses may be used to set and/or optimize therapy treatment dosing for a high-frequency block of a nerve such as the sciatic nerve, dorsal root ganglion (DRG), etc.

For example, described herein are computer-implemented methods determined to be used in treating a patient's pain by delivering neuromodulation (including, but not limited to high-frequency nerve block) from an implanted neuromodulator. Any of these methods may include: receiving a pre-delivery pain estimate from the patient; gathering a set of instantaneous dose parameters in the neuromodulator, wherein the instantaneous dose parameters are provided by a dose selector based on a patient-specific dosing database of previously delivered doses and corresponding estimates of a change in patient-reported pain levels from before and after delivery of each dose; delivering an instantaneous dose using the set of instantaneous dose parameters to the patient from the implanted neuromodulator, wherein the patient or the patient's caregiver may adjust the dose parameters during delivery; receiving a post-delivery pain estimate from the patient within a predetermined time following delivery of the instantaneous dose; adding the pre-delivery pain estimate, the post-delivery pain estimate, the dose instantaneous parameters, and any adjustments made to the dose parameters made during delivery to the patient-specific dosing database; and determining, in a dose selector, a subsequent dose using the patient-specific dosing database. The dose selector may be referred to herein as a dose optimizer.

The pre-delivery and/or post-delivery pain estimate from the patient may be self-reported. For example, the pre-delivery and/or post-delivery pain estimate may be entered by the patient before delivering the instantaneous dose. In some variations, the patient must enter the pre-delivery pain estimate before the dose will be delivered, and/or the system may automatically prompt the user to enter a post-delivery pain estimate. Alternatively or additionally, the pre-delivery pain estimate may be automatically detected by detecting one or more metrics associated with the patient's pain, including heart rate, blood pressure, skin conduction, biomarkers (e.g., cortisol, etc.). In some variation an automatic detection of pain may be made instead or in addition to a patient-reported pain indication.

In some variations a pre-delivery and/or post-delivery pain estimate may be determined by examining one or more motion sensors worn by the patient, and/or implanted in the patient (e.g., in the neuromodulation). For example, the neuromodulator may include one or more motion sensors and motion data may be used to determine an estimate of patient pain; changes in gross movement, small movements, rates of movement, and/or range of movement may be compared for some time period before the dose (pre-dose) and within some time range after the delivery of the dose (post-dose), normalized for time of day/date of week, etc.

In some variations, the patient may be asked or required to perform one or more movements (or a series of movements) before and/or after delivery of a treatment dose, and movement (e.g., motion sensor) and/or vital sign (heart rate, blood pressure, etc.) data taken immediately before, during and/or after the movement may be used to automatically detect a pain indicator.

In some variations, automatic or semi-automatic pain scores may be estimated from one or more images of the patient's face. For example, factors for automatically determining pain intensity for the normalized appearance of the patient's face may be detected by recording an image or video of the patient's face and processing the image(s) or video to determine an estimate of pain level(s).

The pre-delivery and post-delivery pain indicator may be entered into the system, including via a user interface that may be displayed on a computing device, including a handheld device (e.g., smartphone, pad, computer, dedicated controller, etc.). In some variations the pre- and post-delivery pain data may be processed (received, stored, analyzed, modified, etc.) in or partially in the implant (e.g., in a controller in the implant, including a processor, memory, etc.). In some variations the pre- and/or post-delivery pain data may be processed completely or partially in an external component that communicates with the implantable portion. For example, an external patient controller may include software (e.g., an application software), firmware and/or hardware that receives and/or processes the pre-delivery and/or post-delivery pain data to form the pre- and/or post-delivery pain estimate.

In some variations the dose selector receives and processes the pre-delivery and/or post-delivery pain data to form the pre-delivery pain estimate and the post-delivery pain estimate. These pain estimates may be quantified, e.g., scaled in a numeric scale, and/or normalized so that they may be directly compared (including subtracted, converted into a ratio, etc.). The pain score may be normalized to one or more factors (e.g., time of day, patient activity level, etc.).

As mentioned, self-reported pain data (pain estimates) may be collected by a user interface and may allow the user to select from a menu of choices, which may be graphical and/or textual (e.g., "no pain", "mild pain", "moderate pain", "heavy pain", "extreme pain", etc.), and/or ranked (pain score between 0-10, 0-100, etc.).

The dose delivered to the patient (treatment dose) may also be referred to as the instantaneous dose, as it is the dose currently being delivered or to be delivered (in that instant). The dose may be any appropriate duration and intensity, and may be characterized by a set of dose parameters. A dose parameter may be any factor that sets or modifies the dose. For example, dose parameters may include one or more of: initial voltage, peak voltage, frequency (e.g., pulse frequency), treatment/dose duration, onset time (e.g., ramp up time), pulse duration (in bursting/pulsed variations), burst duration (in bursting/pulsed variations), pulse shape (e.g., square, triangular, sinusoidal, etc.), biphasic/monophasic (positive and/or negative) carrier frequency (in variations using a carrier frequency), DC offset level (in variations including a DC offset), current level (in variations modulating current), current limit (in variations limiting current), number of electrodes, location of electrodes (in variations having more than one pair of electrodes), etc.

For example, gathering the set of instantaneous dose parameters may include receiving (e.g., in the controller of the neuromodulator and/or in a control, such as a patient controller or patient control software/app communicating with the neuromodulator) a set of dose parameters (e.g., instantaneous dose parameters) such as: peak voltage, a frequency, and a treatment duration. Any appropriate range of values may be used. For example, the peak voltage may be between 0.1 V to 20 V, between 0.1 V to 15 V, between 0.1 V to 12 V, etc.), the pulse waveform may be a biphasic (or monophasic), sinusoidal (or square, triangular, etc.), the frequency may be, e.g., between 1 KHz and 50 KHz (e.g., between about 2 KHz and 50 KHz, between about 5 KHz and 50 KHz, between about 2 KHz and 30 KHz, between about 5 KHz and 30 KHz, between about 5 KHz and 20 KHz, between about 2 KHz and 20 KHz, etc.). High frequency nerve block may refer to, e.g., a frequency of about 4 KHz or more (e.g., about 4 KHz to about 50 KHz, about 4 KHz to about 40 KHz, about 4 KHz to about 30 KHz, about 4 KHz to about 20 KHz, about 4 KHz to about 10 KHz, etc.). Any appropriate duration (e.g., about 5 minutes, about 10 minutes, about 15 minutes, about 20 minutes, about 25 minutes, about 30 minutes, etc.) may be used. The duration may be duration at peak (e.g., following a ramp-up time) or it may be total duration.

In any of the computer-implemented methods and devices described herein the patient may adjust the one or more dose parameters during delivery. The adjustment may include increasing and/or decreasing the intensity (e.g., the voltage, peak voltage, etc.), changing the frequency (e.g., within a predetermined range, e.g., between about 4 KHz to about 50 KHz, about 4 KHz to about 40 KHz, about 4 KHz to about 30 KHz, about 4 KHz to about 20 KHz, about 4 KHz to about 10 KHz, etc.). Adjusting may include allowing the user to adjust (via a user interface or other patient controller), one or more of the dose parameters. In some variation the patient (sometime referred to a user, which may include the patient and/or caregiver such as a nurse, physician, medical technician, etc.) may switch between two or more (e.g., 2, 3, 4, 5, 6, 7, 8, etc.) set of pre-set dose parameters. For example, a patient controller may control allowing the user to switch between two or more sets of parameters; one or both sets may be determined by the dose selector. The different pre-determined sets may be characterized (e.g., marked, labeled, etc.) as "higher" or "lower" intensity doses. One or both doses may be a high-frequency never block dose.

The dose is typically delivered by the implant (which may include one or more implanted electrodes and a controller). The implant may be connected, e.g., via a nerve cuff, etc. to one or more nerves, or nerve bundles.

In general, any of the computer-implemented methods and apparatuses described herein may include transmitting the pre-delivery pain estimate to the dose selector, including delivering the post-delivery pain estimate, the dose instantaneous parameters, and any adjustments made to the dose parameters made during delivery to the dose selector for entry into the patient-specific dosing database. Before or after delivering the dose parameters to the dose selector, a quantified change in patient-reported pain levels from the pre-delivery pain estimate and the post-delivery pain estimate may be determined. In some variations this change may be determined prior to transmitting to the dose selector (and thus delivering the post-delivery pain estimate and the pre-delivery pain estimate may refer to delivering a combined/compound estimate based on both pre- and post-delivery estimates). The estimate may refer to the raw data from which a processed estimate may be determined.

The dose selector may use the patient-specific database to determine one or more (in variations in which the patient may select between different sets of dose parameters) sets of dose parameters. Although in general the patient-specific database may include just data specific to the patient into which the neuromodulator has been implanted, in some variations the database may initially include one or more start values (starting parameter sets), that may be specific to the patient or may be generic.

The dose selector may use the patient-specific database to select the parameters. For example, the patient specific database may provide multiple sets of parameters and some or all of these parameter sets may include a patient-specific change in pain estimates based on pre- and post-delivery pain estimates. The patient-specific database may also include additional data specific to the patient when the indicated patient-specific change in pain estimates were measured, such as the corresponding time of delivery (time of day), date delivered, corresponding activity level (based on one or more accelerometers), corresponding set(s) of electrodes used, the type of pain estimate (e.g., self-reported and/or automatically reported), the corresponding date of week, etc. Thus, in some variations different pain estimates may be recorded for similar parameter sets taken at different times, etc.

In general, the dose selector may use the patient-specific dosing database to identify an optimal set of parameters. This optimal set may be based on the set having the greatest reduction in pain from pre-treatment and post-treatment that is closest in time (date, and/or time of day, etc.) to the patient. The historical dose treatment parameters may be modified by the dose selector. For example, one or more of the dose parameters (e.g., peak voltage, duration, ramp-on duration, frequency, etc.) may be modified or adjusted. The adjustment, which may be referred to as a "wobble" amount, may be the change (e.g., +/- the parameter value) for one or more of the parameters. The wobble amount may be manually set (e.g., +/- *X%,* where Xis 0.5, 1, 1.5, 2, 3, 4, 5, 6, 7, 8, 9, 10, 15, 20, 30, 35, etc.). In some variations the wobble amount is a variable that may be adjusted by the dose selector (increased or decreased) depending on the difference between the change in patient-reported pain levels compared to prior treatments. Thus, the wobble may be varied by the dose selector to adjust one or more parameters in order to maximize the reduction in patient reported pain level. For example, in some variations, the dose selector may set the wobble amount by identifying a trend in the one or more dose parameters from the patient-specific dosing database and basing the wobble amount on this trend.

Any of the computer-implemented methods and apparatuses described may include a dose selector that is configured to be trained on the patient-specific database using machine learning to determine the wobble and/or the subsequent dose parameters.

For example, the dose selector may determine the subsequent dose(s) using the patient-specific dosing database by identifying a set of prior dose parameters from the patient-specific dosing database having a large reduction in patient-reported pain levels from before and after delivery using the prior dose parameters and modifying one or more of the dose parameters by a wobble amount in the set of prior dose parameters to form a set of subsequent dose parameters that may be delivered as the subsequent dose. The large reduction may be a maximal reduction, or a maximal reduction in a similar set of parameters (e.g., similar pre-treatment pain level, similar activity level, similar time of day, etc.). The large reduction may be greater than a minimum reduction in pain level from pre-treatment to post-treatment (e.g., greater than 10%, greater than 15%, greater than 20%, greater than 30%, greater than 35%, etc.). The reduction in pain level may be an absolute reduction in estimated pain level or it may be a relative reduction in pain level, e.g., normalized to the initial pain level, etc.

A computer-implemented method determined to be used in treating a patient's pain by delivering by neuromodulation from an implanted neuromodulator may include: receiving a pre-delivery pain estimate from the patient; gathering a set of instantaneous dose parameters in the neuromodulator, wherein the instantaneous dose parameters are either manually determined or are provided by a dose selector based on a patient-specific dosing database of previously delivered doses and corresponding estimates of a change in patient-reported pain levels from before and after delivery of each dose; delivering an instantaneous dose using the set of instantaneous dose parameters to the patient from the implanted neuromodulator, wherein the patient or the patient's caregiver may adjust the dose parameters during delivery; receiving a post-delivery pain estimate from the patient within a predetermined time following delivery of the instantaneous dose; adding the pre-delivery pain estimate, the post-delivery pain estimate, the dose instantaneous parameters, and any adjustments made to the dose parameters made during delivery to the patient-specific dosing database; and determining, in a dose selector, a subsequent dose having a set of subsequent dose parameters using the patient-specific dosing database.

Also described herein are systems for treating a patient's pain using high-frequency nerve block. Any of these systems may include: an implantable neuromodulator comprising: one or more electrodes configured to apply high-frequency neural modulation (nerve block) to one or more nerves; and a controller configured to apply a treatment dose having a set of treatment dose parameters from the one or more electrodes; and a dose selector comprising: one or more processors; a patient-specific dosing database of previously delivered doses and corresponding estimates of a change in patient-reported pain levels from before and after delivery of each dose; and a memory coupled to the one or more processors, the memory configured to store computer-program instructions, that, when executed by the one or more processors, perform a computer-implemented method comprising: updating the patient-specific dosing database with the pre-delivery pain estimate from the patient, the post-delivery pain estimate from the patient, the set of treatment dose parameters corresponding to the pre-delivery pain estimate and the post-delivery pain estimate, and any adjustments made to the treatment dose parameters by the patient or the patient's caregiver during delivery; and generating the set of treatment dose parameters based on the patient-specific database.

For example, a system for treating a patient's pain using high-frequency nerve block may include: an implantable neuromodulator comprising: one or more electrodes configured to apply high-frequency neural modulation (e.g., nerve block) to one or more nerves; and a controller configured to apply a treatment dose having a set of treatment dose parameters from the one or more electrodes, wherein the controller is configured to adjust the treatment dose parameters during delivery based on patient or patient caregiver input; and a dose selector comprising: one or more processors; a patient-specific dosing database of previously delivered doses and corresponding estimates of a change in patient-reported pain levels from before and after delivery of each dose; and a memory coupled to the one or more processors, the memory configured to store computer-program instructions, that, when executed by the one or more processors, perform a computer-implemented method comprising: receiving a pre-delivery pain estimate from the patient, a post-delivery pain estimate from the patient, a set of treatment dose parameters corresponding to the pre-delivery pain estimate and the post-delivery pain estimate, and any adjustments made to the treatment dose parameters by the patient or the patient's caregiver during delivery; updating the patient-specific dosing database with the pre-delivery pain estimate from the patient, the post-delivery pain estimate from the patient, the set of treatment dose parameters corresponding to the pre-delivery pain estimate and the post-delivery pain estimate, and any adjustments made to the treatment dose parameters by the patient or the patient's caregiver during delivery; generating a new set of treatment dose parameters based updated patient-specific database; and transmitting the new set of treatment dose parameters to the controller of the implantable neuromodulator.

In general, the systems may be configured to implement any of the computer-implemented methods described herein. These systems may include hardware, software and/or firmware allowing the performance of any of the computer-implemented methods.

For example, the system may include an implant (e.g., implantable neuro modulator) and/or a patient controller. The patient controller may be external to the patient and may allow the user (e.g., patient and/or caregiver, such as physician, nurse, medical technician, etc.) to control some aspects of the implantable neuromodulator. The patient controller may include the dose selector and may include the patient-specific database.

Any of these systems described herein may include a user interface (e.g., as part of a patient controller) that is configured to allow patient entry of the pre-delivery pain estimate and the post-delivery pain estimate.

The dose selector may be part of a patient controller and/or part of a remote server. In some variations the dose selector may be in or part of the implant, particularly (but not limited to) variations in which the pre-treatment and/or post-treatment pain estimates are determined automatically or semi-automatically (e.g., from motion data). The dose selector may include one or more processors (which may be related to or separate from the processor(s) of the patient controller), and one or more memories, which may include or be part of the patient-specific database. The patient-specific database may be maintained locally (e.g. as part of the patient controller and/or implant) and/or remotely (as part of the remote server). The patient-specific database may be redundant between one or more of the patient controller, remote database and/or implant. The dose selector may include logic (e.g., software or firmware) for performing all or part of any of the computer-implemented methods described herein, including determining wobble and selecting and/or modifying dose parameters from the patient-specific database to determine the parameters for subsequent dose(s) (or dose options).

The systems may be configured to automatically get the pre-treatment pain data and/or post-treatment pain data. For example, the patient controller may include a user interface for receiving user-reported pain data. In some variations the system may require the user to enter pre-treatment pain data. For example, any of these systems may be configured to prevent the controller from applying the treatment dose until the patient has entered the pre-delivery pain estimate into the user interface. In some variations the system may be configured to automatically prompt the patient for the post-delivery pain estimate a predetermined time after the treatment dose has been applied.

The controller (e.g., patient controller) may be configured to adjust the treatment dose parameters during delivery based on patient or patient caregiver (e.g., user) input. For example, the controller may be configured to allow the user to select between two or more sets of treatment parameters.

The computer-implemented method of the dose selector may be configured to perform any of the steps of the method described herein. For example, generating the set of treatment dose parameters based on the patient-specific database may comprise generating a peak voltage, a frequency, and a treatment duration; for example, this may include identifying a set of prior dose parameters from the patient-specific dosing database having a high scoring change in patient-reported pain levels from before and after delivery using the prior dose parameters; and modifying one or more of the prior dose parameters by a wobble amount in the set of prior dose parameters to form the set of treatment dose parameters. As mentioned, the wobble amount may comprise a percentage of the value of the one or more dose prior parameters. The system may set the wobble amount based by identifying a trend in the one or more dose parameters from the patient-specific dosing database.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 shows one example of a neuromodulation system (showing a nerve cuff, lead and implantable controller/waveform generator).
FIG. 2A shows an example of the system of FIG. 1 implanted into a patient (also showing a controller (in this example, an external controller) for controlling and applying a therapeutic dose. The external controller (also referred to herein as a patient controller or user controller) may include or communicate with a dose selector.
FIG. 2B shows an example of a system for treating a patient's pain using high-frequency nerve block that includes a dose selector as described herein.
FIG. 2C schematically illustrates one example of a system for treating a patient's pain including a dose selector.
FIG. 2D schematically illustrates one example of a dose selector (e.g., configured as a dose selector module) that may be used as part of a system such as the system of FIG. 2C.
FIG. 3A is an example of a schematic of a voltage profile of a therapeutic dose.
FIG. 3B illustrates examples a schematic of the voltage profiles of two therapeutic doses that may be applied by an implanted neuromodulator as described herein.
FIG. 3C illustrates another example of a voltage profile of a therapeutic dose.
FIG. 4 is a flow diagram illustrating one example of a method of treating a patient's pain by delivering neuromodulation from an implant using patient-specific dose setting (e.g., optimization).
FIG. 5A is a first example of a user interface (e.g., of a user control) for entering the patient's current pain level.
FIG. 5B is a second example of a user interface for entering the patient's current pain level.

### DETAILED DESCRIPTION

In general, the computer implemented methods and apparatuses for performing them described herein allow dose setting (including optimized dose setting) of a neuromodulation apparatus so that the therapy dose provided by the neuromodulator maximizes the energy which may enhance the effect of the neuromodulation on the target nerve(s) without irritating or harming the patient. The computer-implemented methods and apparatuses may be generally described for use with an implanted neuromodulator, but may be also or alternatively be used with external neuromodulators or neuromodulators prior to implantation. Further, the examples provided herein are provided in reference to neuromodulatory inhibition by the application of high-frequency neuromodulation, however the computer-implemented methods and apparatuses may also be used with other neuromodulatory regimes including general neuromodulation. Examples of neuromodulator apparatuses and computer-implemented methods that may benefit from the computer-implemented methods and apparatuses may include, for example, spinal cord stimulators (SCS) and any other neuromodulation application that may be improved by the optimization between therapeutic benefit and induced sensation, including reducing a patient's pain.

The inventors have generally found that increasing the applied voltage of neuromodulation is beneficial, particularly when sustained at a high voltage (e.g., high peak voltage). However, the efficacy of the neuromodulation to treat, e.g., pain may vary between individuals and even in the same individual over time. Further, some neuromodulation doses, and particularly those at relatively high voltage, which may be applied to a patient's nerve as part of a high-frequency nerve block to treat pain, may be less effective depending on the dosing parameters and may instead result in discomfort for the patient in some cases, even for dose parameters that may be effective for other patients. For example, if the applied peak voltage exceeds a threshold voltage during the ramp up to the sustained high voltage, the patient may experience discomfort or even pain during treatment. The value of this threshold may vary between patients and also appears to vary based on the prior neuromodulation already experienced by the nerve as well as the modulation parameters (e.g., frequency).

Described herein are computer-implemented methods and apparatuses (e.g., systems, devices, and software, including neuromodulators and neuromodulation systems) for treating a patient's pain that include a dose selector that may learn and adapt to a specific patient's response to neuromodulation so as to provide dose parameters that more effectively reduce or eliminate pain in the patient. These computer-implemented methods and apparatuses may, over time, become more effective at treating the patient's pain.

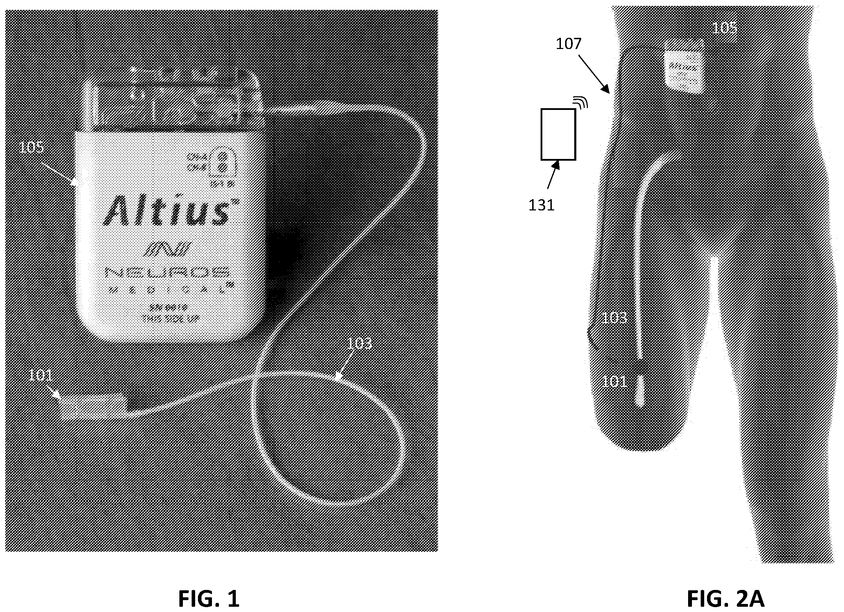

These computer-implemented methods and apparatuses may be used with any appropriate neuromodulator. FIG. 1 illustrates one example of an implantable neuromodulator including a nerve cuff 101, a lead 103 connecting the nerve cuff to a controller (e.g., waveform generator, control circuitry, power source, communications circuitry and/or antenna, etc.) 105. Systems including a nerve cuff such as those described herein, may be used, for example, to apply a high frequency nerve block to acutely treat pain, either acute pain or chronic pain (more than 6 months in duration), in humans by blocking nerve conduction on an action potential. Acute treatment may refer to on-demand treatment with substantially immediate pain relief effect. The nerve cuff may be applied onto a moderate and relatively large diameter nerves such as the sciatic nerve. One therapy involves reversibly blocking peripheral nerves by applying high frequency alternating current directly on a nerve trunk. Specifically, a current ranging from 1 kHz to 100 kHz (e.g., 5 kHz to 50 kHz) may be applied; this may be referred to as a high frequency modulation, compared to a current of less than 1 kHz applied in the conventional electrical modulation described above. Efficacy of the high frequency alternating current therapy in acute non-human animal experiments (frog, cat) has been reported. U.S. Pat. Nos. 7,389,145 and 8,060,208 describe in general this electrical modulation technology.

The nerve cuffs may encircle a particular segment of a targeted peripheral nerve, e.g., a sciatic nerve, a tibial nerve, etc. Using an implanted electrode connected to an electrical waveform generator, an electrical waveform may be applied for a time interval, e.g., 10 min (15 min, 20 min, 25 min, 30 min, 35 min, 40 min, etc.), sufficient to effect substantially immediate patient pain relief, e.g., within 10 min, and an extended period of pain relief up to several hours. The current may range, for example, from 4 mApp to 26 mApp.

The application of 10 kHz alternating current generated by a custom generator via a custom implanted nerve electrode may significantly reduce pain in the majority of patients treated. For example, an implantable electrode operatively connected to an external or implanted waveform generator may be used. The electrode may be a spiral cuff electrode similar to that described in U.S. Pat. No. 4,602,624. The electrode may be implanted in a human mammal on a desired peripheral nerve trunk proximal to the pain source (e.g., a neuroma), such that the cuff encircled the desired peripheral nerve in which the action potential was to be blocked. The cuff inner diameter may range from about 4 mm to about 13 mm. The sciatic nerve is known to have a relatively large nerve trunk; the diameter of the proximal part of the sciatic nerve in a human adult is about 12 mm. In one embodiment, the apparatus and method was used on the sciatic nerve to treat limb pain in above knee amputees. In one embodiment, the apparatus and method was used on the tibial nerve to treat limb pain in below knee amputees.

FIG. 2A illustrates the use of a system including a cuff electrode applied to the sciatic nerve of an amputee patient. In this example, the amputee 107 has been implanted with a nerve cuff 101 around the sciatic nerve (nerve trunk), and is connected, via a lead 103, to the controller including the waveform generator 105. This procedure may be done, for example, by first dissecting to expose the nerve in an open procedure, then wrapping the nerve with the flexible (self-closing) cuff. Once implanted the controller/waveform generator may be placed in a pocket in the anterorlateral abdominal wall, and a tunneling electrode cable may be positioned along the midaxilalary line (including transversely across the abdomen) to connect the controller/waveform generator to the nerve cuff electrode. Once the impedance of the nerve cuff is checked (e.g., by the controller) the incisions may be closed. The incision for implanting the nerve cuff is typically larger than about 1.5 inches (e.g., between 1.5 and 3 inches), so that sufficient visualization and access may be achieved. Once implanted and allowed to heal, the implanted neuromodulator may be set as described herein to provide a therapeutic dose (e.g., an optimized dose) as described herein.

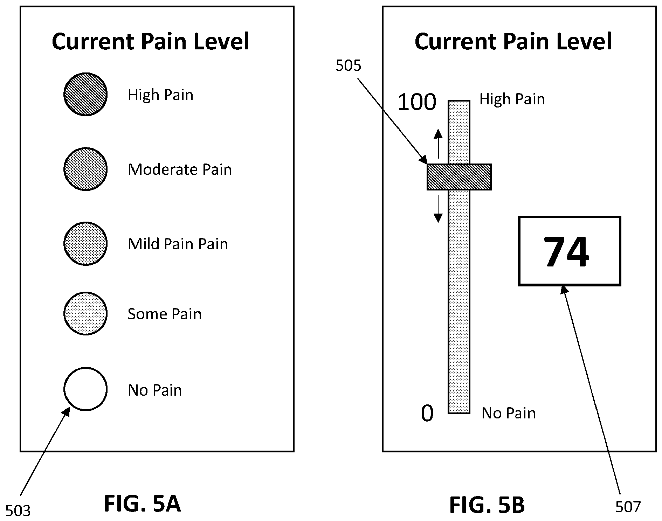

The system shown in FIG. 2A also includes a controller 131, shown as an external controller (e.g., patient controller) that include one or more processors and may be configured to perform the methods described herein. The controller, or a separate device coupled to the controller, may include an input for the user to report pain data, such as the pre-treatment and/or post-treatment pain. The controller may be software (such as application software) that runs on a personal device, such as an smartphone, table, etc., and may include one or more user interfaces to allow the user to enter the pain data. See, e.g., FIGS. 5A and 5B, illustrating user interfaces for a controller that allows the user to enter their current pain level. In FIG. 5A the current pain level is entered by selecting from five options (no pain, some pain, mild pain, moderate pain, and severe pain). A visual cue 503 may be used and may be a "button" to allow the user to select the pain level, as shown in FIG. 5A. In some variations an icon (e.g., smiling face to frowning face) may be used. In FIG. 5B, the input is a slider 505 for selecting the level of pain between 0 (no pain) and 100 (severe pain). The pain score 507 may also be shown and/or may be manually entered. Other user interfaces may be used.

A neuromodulation dose may include a variety of dose parameters for treating pain. In general, a set of dose parameters may include a dose duration (e.g., time the dose is being delivered, which may be the total duration or may be a portion of the total duration, including both on time, e.g., ramp-up time and treatment time, e.g., time a peak voltage), dose frequency (e.g., treatment frequency; in high-frequency never block variations the frequency may be greater than 1 KHz, such as between 1-100 KHz), and peak voltage (e.g., peak modulation voltage, such as between 0.1 V and 20 V, e.g., between 5 V and 15 V, etc.). In some variation the dose parameters may include a therapy ramp-up time to reach a peak modulation voltage and a sustained peak modulation time during which the voltage is sustained at the peak modulation voltage (the dose duration may include both ramp-up time and peak modulation time). The dose parameters may also include the waveform parameters applied, e.g., pulsatile or repeating (e.g., sinusoidal, square wave, saw-tooth, biphasic, etc.), and the frequency of the applied waveform (e.g., the high-frequency component). Other dose parameters may include the initial (e.g., starting) voltage, which may be, e.g., zero, or may be an offset (e.g., voltage offset) voltage. In some variations, the therapeutic dose parameters may include pulse duration (in treatment variations including bursting/pulses), burst duration (in variations including bursting/pulses), pulse shape (e.g., square, triangular, sinusoidal, etc.), biphaic/monophasic (positive and/or negative), carrier frequency (in variations using a carrier frequency), DC offset level (in variations including a DC offset), current level (in variations modulating current), current limit (in variations limiting current), electrode number/location (in variations having more than one pair of electrodes), etc.

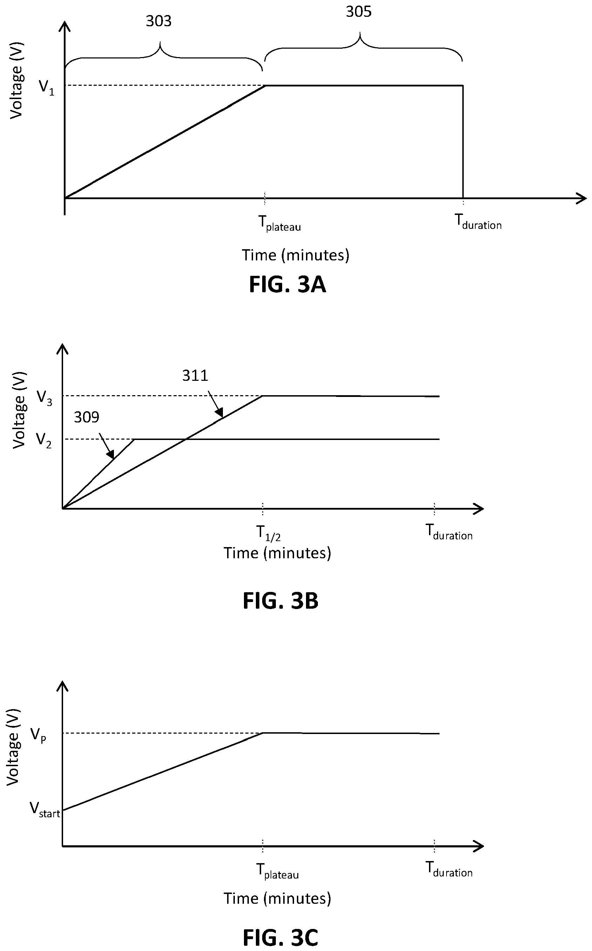

FIG. 3A illustrates one example of an exemplary voltage profile of a therapeutic dose. In this example, the first portion of the therapeutic dose is a ramp-up period 303, which has a duration (referred to as a therapy ramp-up time to reach a peak modulation voltage, Vi in FIG. 3A) of Tₚₗₐₜₑₐᵤ that is between 10% and 90% of the total duration of the therapeutic dose (T_{duration}). The second portion of the therapeutic dose is a sustained peak modulation time 305 during which the voltage is sustained at the peak modulation voltage (V₁), this may also be referred to as the plateau portion.

During the ramp-up portion of the therapeutic dose, the neuromodulator may apply an increasing intensity of modulation from the start (time 0, T₀) to the peak modulation voltage (Vi) at time Tₚₗₐₜₑₐᵤ. In FIG. 3A this ramp period is shown as a linear increase, however it may increase in steps (e.g., incrementally increase following regular interval, such as increasing by 0.5 V every 30 seconds, etc.). In practice, the modulation may be applied with a high-frequency component having a frequency of between about 1 kHz and 100 kHz (e.g., 1 kHz and 50kHz, 1 kHz and 40 kHz, 1 kHz and 30 kHz, 1kHz and 25 kHz, about 5 kHz, about 10 kHz, etc.).

FIG. 3B illustrates another example of a pair of therapeutic dose profiles. In one example 309, a rapid ramp up reaches a first voltage level (V₂), and is held at this first voltage level for the duration of the dose. This first voltage level may result in a first sensation intensity for the patient, including a sensation intensity that is the maximum sensation that can be tolerated for the dose. The second profile 311 shows an example in which an approximately equivalent sensation intensity for the patient may be experienced, but with a much high voltage (V₃) that is reached by ramping up over a longer time period (shown as T_{1/2}, approximately half of the duration of the dose, T_{duration}). In this example, although the time at which the modulation applied is at the maximum peak voltage is longer for the first profile, the second profile has a much higher maximum peak voltage. In practice, once a maximum peak voltage is determined, a dose profile may be set for the neuromodulator by using a starting voltage (which may be determined from the minimum patient-detectable modulation voltage detectable by the patient) the peak modulation voltage (which may be determined as described herein) the ramp-up time to reach the peak modulation voltage, and either or both the total duration of the dose and/or the plateau duration, or equivalent values from which these values may be derived. The ramp-up time to reach the peak modulation voltage may be expressed as a time, or as a rate of voltage increase. The therapeutic dose may also include the waveform parameters to be applied (e.g., pulsed, such as sinusoidal), and the high-frequency component (e.g., between 1 kHz and 100 kHz, between 1 kHz and 50 kHz, e.g., about 5 kHz, about 10 kHz, etc.), etc.

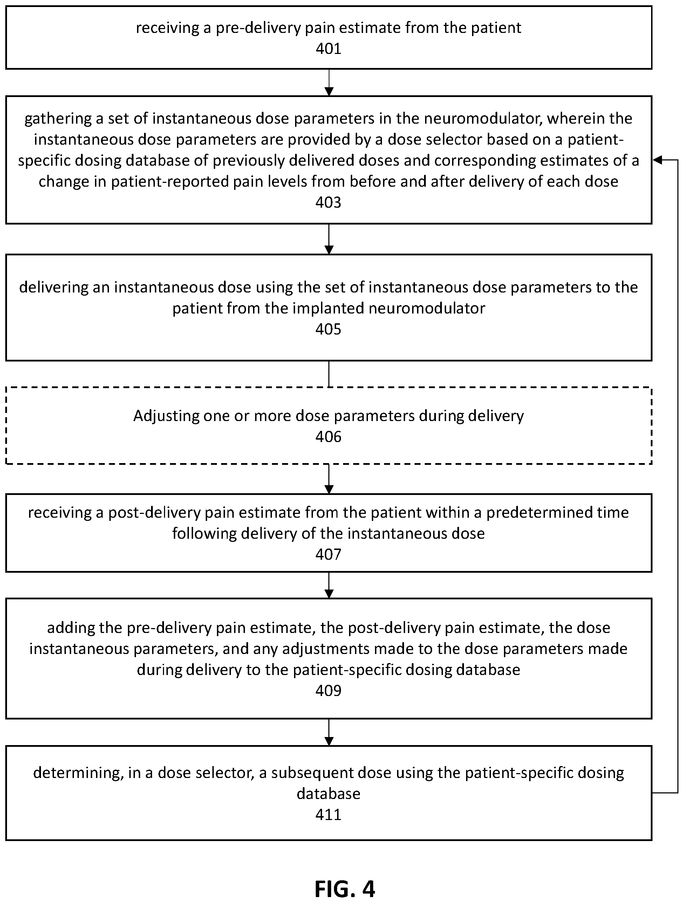

FIG. 4 describes a general computer-implemented method of treating a patient's pain by delivering neuromodulation from an implanted neuromodulator that includes a dose selector. In FIG. 4, a pain level for the patient may be taken prior to deliver of a treatment dose 401. In some variations the patient, who typically has an implanted neuromodulator, may first enter an estimate of their current pain level (e.g., pre-delivery pain estimate), before the start of a treatment dose. For example, using a user interface on the patient controller (e.g., a user interface such as those shown in FIGS. 5A-5B).

Alternatively or additionally, in some variations the apparatus may interrogate the patient indirectly, by monitoring patient biometric information (heart rate, pulse, blood pressure, ensemble nerve activity, skin conductance, respiration, biomarker, including pain biomarker, levels, etc.) that may also be correlated with this applied ramp to determine a target sensation intensity modulation voltage, including a maximum patient-tolerable modulation voltage. In some variations a proxy for user pain may be estimated base on patient movement. For example, the system may include one or more accelerometers, including as part of the neuromodulator(s) that measure patient movement(s). Accelerometer information, including tremors (e.g., higher frequency movements), reduction in overall movement levels and/or reduction in the range of movement(s) may indicate pain level. The system may include a pain detection module that may estimate pain based on the accelerometer information from a period of time (e.g., 1 minute, 2 minutes, 3 minutes, 5 minutes, 10 minutes, etc.) prior to delivery of the therapeutic dose. In some variations the patient may be asked to perform one or a sequence of particular movement(s), e.g., moving the portion of the body including the neuromodulator, or gross movements, during the time period in which pain is to be estimated.

In some variations, pain may be estimated from the patient indirectly by taking a picture, series of picture or video of the patient's face during this time period and used to estimate pain based on an analysis of the patient's expression(s). See, e.g., "Facial Expression Analysis for Estimating Pain in Clinical Settings" K. Sikka, (conference paper) ICMI' 14, November 12-16, Istanbul, Turkey. For example, the patient may be asked to take an image or video (e.g., using their smartphone) of their face for a predetermined time to estimate pain, including self-imaging while performing one or more movements.

Both direct (patient self-reported) and/or indirect pain data may be used to determine an estimate of pain for the patient. In some variations, the delivery of the treatment dose may require receipt of the pre-delivery pain data (e.g., pain estimate).

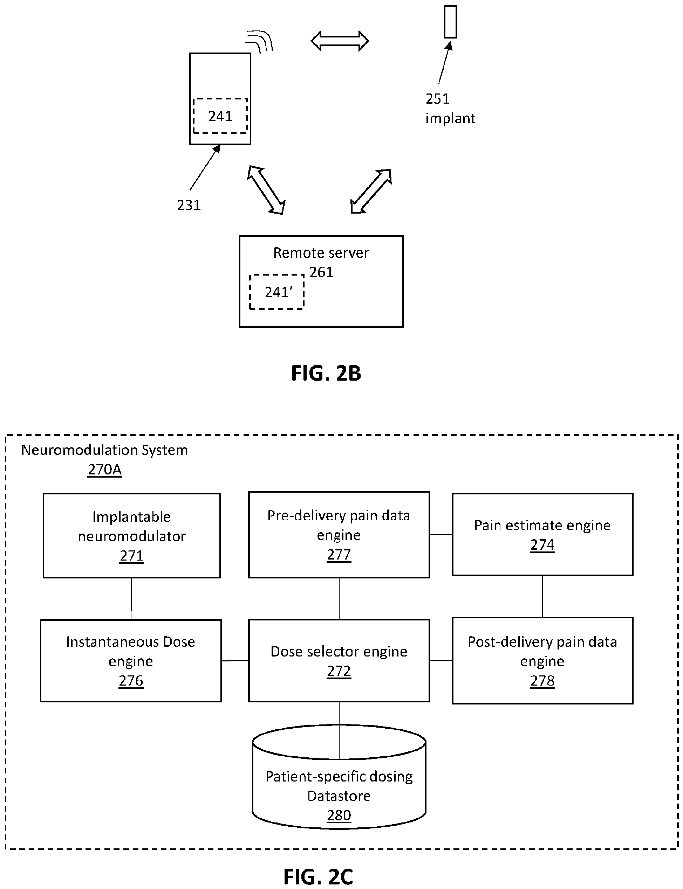

Thereafter, the computer-implemented method and apparatus may then gather a set of instantaneous dose parameters in the neuromodulator 403. The instantaneous dose parameters may be provided by a dose selector and/or they may be manually set. For example the dose selector may provide one or more sets of instantaneous dose parameters that are based on a patient-specific dosing database of previously delivered doses and corresponding estimates of a change in patient-reported pain levels from before and after delivery of each dose. For example the dose parameters may be based on known "effective" dose parameters based on the improvement in pain estimates from pre-treatment and post-treatment pain estimates of previously delivered treatment doses in the patient-specific database. The patient-specific database 241, 241' may be part of the local (patient controller 231) and/or part of a remote processor (e.g., remote server 261) accessed by the neuromodulator and/or patient controller, as shown in FIG. 2B.

The dose selector may select a dose from the patient-specific database or, if no patient-specific data is available yet, it may select a starting dose; the dose selector may then modify one or more of the dose parameters in order to improve on the dose efficacy. For example, in some variations one or more dose parameters may be adjusted by a wobble amount (e.g., +/- a percentage of the value of the dose parameter). The dose selector may be trained from the patient-specific database to select or estimate treatment parameters in order to maximize the reduction in pain, particularly in the current context (e.g., time of day, time since last treatment, patient HR, patient pre-treatment pain score, etc.). In some variations a 'high' treatment dose (more aggressive, e.g., higher voltage, longer time, shorter on-time, etc.) may be included along with a second (or more) 'lower' treatment dose(s) (e.g., less aggressive, lower voltage, longer on-time, etc.), so that the patient may switch between the two during treatment.

Returning to FIG. 4, once one or more instantaneous doses have been provided, the treatment dose (instantaneous dose) may be applied using the set of instantaneous dose parameters, from the implanted neuromodulator 405. In some variations, the patient or the patient's caregiver may adjust the dose parameters during delivery 406. For example, one or more dose parameters may be manually adjusted within a predetermined range (e.g., peak voltage, etc.), and/or the user (patient or caregiver) may select between two or more sets of treatment parameters. Any modifications to the dose parameters may be recorded by the system and later transmitted to the patient-specific database along with the pre- and post-treatment pain data.

After some time, which may be set by the system, following treatment, a second (post-delivery) pain estimated may be entered 407. For example, the patient (e.g., via the patient controller) may be prompted to enter a self-reported pain estimate, and/or pain data may be automatically or semi-automatically entered as described above.

The applied treatment parameters (including any user modifications) and the pre-treatment and post-treatment pain data (e.g., pain estimates) may then be transmitted to and stored in the patient-specific database 409 for access by (and or training of) the dose selector. For example, the dose selector may then be used to determine any subsequent dose(s) 411 using the patient-specific dosing database. This process may be iteratively repeated with each application of a treatment dose. With each iteration or repetition, the dose selector may converge on more optimal treatment parameters, even as the patient's tolerance changes over time.

### Example Structures and Systems

FIG. 2C is a diagram showing an example of a neuromodulation system that include dose setting as described above 270A. The modules of the neuromodulation system 270A may include one or more engines and datastores. A computer system can be implemented as an engine, as part of an engine or through multiple engines. As used herein, an engine includes one or more processors or a portion thereof. A portion of one or more processors can include some portion of hardware less than all of the hardware comprising any given one or more processors, such as a subset of registers, the portion of the processor dedicated to one or more threads of a multi-threaded processor, a time slice during which the processor is wholly or partially dedicated to carrying out part of the engine's functionality, or the like. As such, a first engine and a second engine can have one or more dedicated processors or a first engine and a second engine can share one or more processors with one another or other engines. Depending upon implementation-specific or other considerations, an engine can be centralized or its functionality distributed. An engine can include hardware, firmware, or software embodied in a computer-readable medium for execution by the processor. The processor transforms data into new data using implemented data structures and computer-implemented methods, such as is described with reference to the figures herein.

The engines described herein, or the engines through which the systems and devices described herein can be implemented, can be cloud-based engines. As used herein, a cloud-based engine is an engine that can run applications and/or functionalities using a cloud-based computing system. All or portions of the applications and/or functionalities can be distributed across multiple computing devices, and need not be restricted to only one computing device. In some embodiments, the cloud-based engines can execute functionalities and/or modules that end users access through a web browser or container application without having the functionalities and/or modules installed locally on the end-users' computing devices.

As used herein, datastores are intended to include repositories having any applicable organization of data, including tables, comma-separated values (CSV) files, traditional databases (e.g., SQL), or other applicable known or convenient organizational formats. Datastores can be implemented, for example, as software embodied in a physical computer-readable medium on a specific-purpose machine, in firmware, in hardware, in a combination thereof, or in an applicable known or convenient device or system. A database may be a datastore or part of a datastore. Datastore-associated components, such as database interfaces, can be considered "part of" a datastore, part of some other system component, or a combination thereof, though the physical location and other characteristics of datastore-associated components is not critical for an understanding of the techniques described herein.

Datastores can include data structures. As used herein, a data structure is associated with a particular way of storing and organizing data in a computer so that it can be used efficiently within a given context. Data structures are generally based on the ability of a computer to fetch and store data at any place in its memory, specified by an address, a bit string that can be itself stored in memory and manipulated by the program. Thus, some data structures are based on computing the addresses of data items with arithmetic operations; while other data structures are based on storing addresses of data items within the structure itself. Many data structures use both principles, sometimes combined in non-trivial ways. The implementation of a data structure usually entails writing a set of procedures that create and manipulate instances of that structure. The datastores, described herein, can be cloud-based datastores. A cloud-based datastore is a datastore that is compatible with cloud-based computing systems and engines.

The neuromodulator system 270A may include a computer-readable medium, an implantable neuromodulator 271, and one or more automated dose selecting (e.g., dose optimization or dose setting) engine(s) 272, instantaneous dose engine(s) 276, pre-pain data engine 277, post-pain data engine 278, and pain estimate engine(s) 274, as well as the patient-specific dosing datastore 280. Functionally, the implantable neuromodulator may be implanted in the patient (as shown in FIGS. 2A-2B) and may communicate with the other components of the system. For example, the instantaneous dose engine 276 may provide the one or more sets of dose treatment parameters to the implant for application to the patient. The dose engine 276 may also allow the patient to adjust the dose, including switching to a different set of dose parameters, as described above. In some variations the dose engine 276 is integrated into or part of the patient controller.

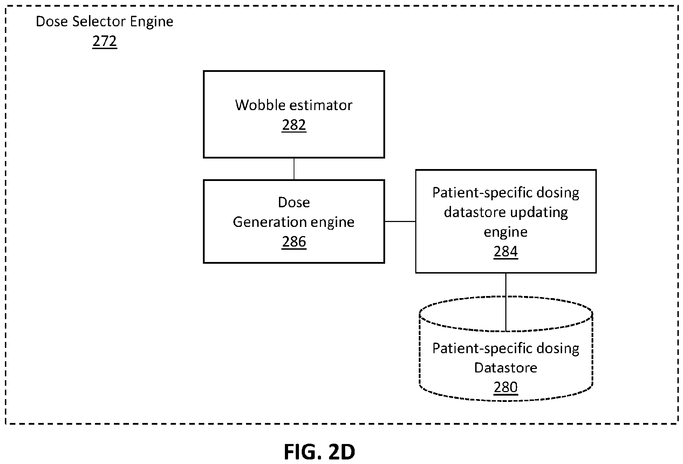

The dose selector (e.g., dose optimization engine) 280 may perform the setting (e.g., optimization) of the dose treatment parameters described herein. The dose selector engine 272 is shown schematically in FIG. 2D, and may include a dose generation engine 286 (e.g., subsequent dose parameter generation engine), which may use a wobble estimator 282 to modify one or more sets of dose parameters from the patient specific dosing datastore 280. The patient-specific dosing datastore (e.g., database) may be updated and/or maintained by the patient-specific dosing datastore updating engine 284. This updating engine may receive input from the pain estimation engine(s) 274, 277, 278.

In any of the computer-implemented methods and apparatuses described herein, the computer-implemented method may include verification of the patient-reported pain data, as well as any of the data (e.g., time of day, date, etc.) to be stored in the patient-specific database. In some cases, patient-specific data may include errors or may be less reliable because of variability in patient reporting, or erroneous entry of data. For example, the date and time information associated with a treatment may be incorrect if the patient controller (e.g., smartphone, dedicated controller, etc.) includes an incorrect date/time (e.g., because of a power failure, or the like).

Data verification may include checking or comparing data, including time/date and patient self-reported data, to a remote database (e.g., server), and making corrections based on verified data on the server. For self-reported pain data, the data may be verified against automatically or semi-automatically collected data (e.g., HR, blood pressure, facial expression, movement data, etc.). In some variations the self-reported patient data may alternatively or additionally be verified and/or weighted by a verification engine that may compare a particular pre- and/or post-treatment self-reported pain data against historical data, e.g., in the patient-specific database to track self-reported responses over time. The verification engine may examine the trend of self-reported data and determine outliers (e.g., look for randomness) and may apply a confidence value (e.g., weighting) to data that appears less or more reliable. In some variations the user interface may also be used as part of the verification engine, which may look, for example, at the length of time taken for entering the data (e.g., time to respond); responding too fast or too slow may decrease the confidence weighting. Thus, the patient-specific database may include confidence weightings for the pre- and/or post-treatment pain data (estimates).

Any of the computer-implemented methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to control perform any of the steps, including but not limited to: displaying, communicating with the user, analyzing, modifying parameters (including timing, frequency, intensity, etc.), determining, alerting, or the like.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of" or alternatively "consisting essentially of' the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. A system for treating a patient's pain using high-frequency nerve block, the system comprising:
an implantable neuromodulator (271) comprising:
one or more electrodes configured to apply high-frequency neural modulation to one or more nerves; and
a controller (105, 131, 231) configured to apply a treatment dose having a set of treatment dose parameters from the one or more electrodes; and
a dose selector (280) comprising:
one or more processors;
a patient-specific dosing database (409) of previously delivered doses and corresponding estimates of a change in patient-reported pain levels from before and after delivery of each dose; and
a memory coupled to the one or more processors, the memory configured to store computer-program instructions, that, when executed by the one or more processors, perform a computer-implemented method comprising:
updating the patient-specific dosing database (409) with a pre-delivery pain estimate from the patient, a post-delivery pain estimate from the patient, the set of treatment dose parameters corresponding to the pre-delivery pain estimate and the post-delivery pain estimate, and any adjustments made to the set of treatment dose parameters by the patient or the patient's caregiver during delivery; and
generating the set of treatment dose parameters based on the patient-specific dosing database (409).

2. The system of claim 1, further comprising a user interface configured to allow patient entry of the pre-delivery pain estimate and the post-delivery pain estimate.

3. The system of claim 2, wherein the system is configured to prevent the controller from applying the treatment dose until the patient has entered the pre-delivery pain estimate into the user interface.

4. The system of claim 2, wherein the system is configured to automatically prompt the patient for the post-delivery pain estimate a predetermined time after the treatment dose has been applied.

5. The system of claim 1, wherein the controller is configured to adjust the set of treatment dose parameters during delivery based on patient or patient caregiver input.

6. The system of claim 1, wherein the computer-implemented method further comprises transmitting the set of treatment dose parameters to the controller (105, 231) of the implantable neuromodulator (271).

7. The system of claim 1, wherein generating the set of treatment dose parameters based on the patient-specific database (241, 241', 411) comprises generating a peak voltage, a frequency, and a treatment duration.

8. The system of claim 1, wherein generating the set of treatment dose parameters based on the patient-specific database (241, 241', 411) comprises identifying a set of prior dose parameters from the patient-specific dosing database (409) having a high scoring reduction in patient-reported pain levels from before and after delivery using the prior dose parameters; and modifying one or more of the prior dose parameters by a wobble amount in the set of prior dose parameters to form the set of treatment dose parameters.

9. The system of claim 8, wherein the wobble amount comprises a percentage of the value of the one or more dose prior parameters.

10. The system of claim 8, further comprising setting the wobble amount based by identifying a trend in the one or more dose parameters from the patient-specific dosing database (409).

11. The system of claim 1, wherein the dose selector (280) is part of the implant.

12. The system of claim 1, wherein the does selector is part of a patient controller and/or part of a remote server (261).

13. The system of claim 1, wherein the computer-implemented method is further configured to determine subsequent doses using the patent-specific dosing database (409).

14. The system of claim 1, wherein the computer-implemented method is further configured to receive the pre-delivery pain estimate from the patient, the post-delivery pain estimate from the patient, the set of treatment dose parameters corresponding to the pre-delivery pain estimate and the post-delivery pain estimate, and any adjustments made to the treatment dose parameters by the patient or the patient's caregiver during delivery.

## Patentansprüche

1. System zur Behandlung von Schmerzen eines Patienten unter Verwendung einer Hochfrequenz-Nervenblockade, das System umfassend:
einen implantierbaren Neuromodulator (271) umfassend:
eine oder mehrere Elektroden, konfiguriert, um eine Hochfrequenz Neuromodulation auf einen oder mehrere Nerven anzuwenden; und
ein Steuergerät (105, 131, 231), konfiguriert, um eine Behandlungsdosis anzuwenden, die einen Satz von Behandlungsdosisparametern von der einen oder den mehreren Elektroden hat; und
einen Dosiswähler (280), umfassend:
einen oder mehrere Prozessoren;
eine patientenspezifische Dosierungsdatenbank (409) von zuvor abgegebenen Dosen und entsprechende Einschätzungen von einer Änderung der vom Patienten berichteten Schmerzwerte von vor und nach der Abgabe jeder Dosis; und
einen Speicher, gekoppelt an einen oder mehrere Prozessoren, wobei der Speicher konfiguriert ist, um
Computerprogrammanweisungen zu speichern, die bei Ausführung durch den einen oder die mehreren Prozessoren ein computerimplementiertes Verfahren durchführen, umfassend:
Aktualisieren der patientenspezifischen Dosierungsdatenbank (409) mit einer Schmerzeinschätzung des Patienten vor der Abgabe, einer Schmerzeinschätzung des Patienten nach der Abgabe, dem Satz von Behandlungsdosisparametern, die der Schmerzeinschätzung vor der Abgabe und der Schmerzeinschätzung nach der Abgabe entsprechen, und jedweden Anpassungen, die durch den Patienten oder die Pflegeperson des Patienten an dem Satz von Behandlungsdosisparametern während der Abgabe vorgenommen wurden; und
Erzeugen des Satzes von Behandlungsdosisparametern basierend auf der patientenspezifischen Dosierungsdatenbank (409).

2. System nach Anspruch 1, ferner umfassend eine Benutzerschnittstelle, konfiguriert, um dem Patienten die Eingabe der Schmerzeinschätzung vor der Abgabe und der Schmerzeinschätzung nach der Abgabe zu ermöglichen.

3. System nach Anspruch 2, wobei das System konfiguriert ist, um zu verhindern, dass das Steuergerät die Behandlungsdosis anwendet, bis der Patient die Schmerzeinschätzung vor der Abgabe in die Benutzerschnittstelle eingegeben hat.

4. System nach Anspruch 2, wobei das System konfiguriert ist, um den Patienten nach einer vorbestimmten Zeit, nachdem die Behandlungsdosis abgegeben wurde, automatisch aufzufordern, die Schmerzen nach der Abgabe einzuschätzen.

5. System nach Anspruch 1, wobei das Steuergerät konfiguriert ist, um den Satz von Behandlungsdosisparametern während der Abgabe basierend auf den Eingaben des Patienten oder des Pflegepersonals des Patienten anzupassen.

6. System nach Anspruch 1, wobei das computerimplementierte Verfahren ferner das Übertragen des Satzes von Behandlungsdosisparametern an das Steuergerät (105, 231) des implantierbaren Neuromodulators (271) umfasst.

7. System nach Anspruch 1, wobei das Erzeugen des Satzes von Behandlungsdosisparametern basierend auf der patientenspezifischen Datenbank (241, 241', 411) das Erzeugen einer Spitzenspannung, einer Frequenz und einer Behandlungsdauer umfasst.

8. System nach Anspruch 1, wobei das Erzeugen des Satzes von Behandlungsdosisparametern basierend auf der patientenspezifischen Datenbank (241, 241', 411) das Identifizieren eines Satzes von früheren Dosisparametern aus der patientenspezifischen Dosierungsdatenbank (409) umfasst, die eine hoch bewertete Reduktion der vom Patienten berichteten Schmerzwerte von vor und nach der Abgabe unter Verwendung der früheren Dosisparameter hat; und das Modifizieren eines oder mehrerer der früheren Dosisparameter um einen Abweichungsbetrag im Satz der früheren Dosisparameter, um den Satz von Behandlungsdosisparametern zu bilden.

9. System nach Anspruch 8, wobei der Abweichungsbetrag einen Prozentsatz des Wertes des einen oder der mehreren früheren Dosisparameter umfasst.

10. System nach Anspruch 8, ferner umfassend das Einstellen des Abweichungsbetrags basierend auf dem Identifizieren einer Tendenz in dem einen oder den mehreren Dosisparametern in der patientenspezifischen Dosierungsdatenbank (409).

11. System nach Anspruch 1, wobei der Dosiswähler (280) Teil des Implantats ist.

12. System nach Anspruch 1, wobei der Dosiswähler Teil eines Patientensteuergeräts und/oder Teil eines Remote-Servers (261) ist.

13. System nach Anspruch 1, wobei das computerimplementierte Verfahren ferner konfiguriert ist, um nachfolgende Dosen unter Verwendung der patientenspezifischen Dosierungsdatenbank (409) zu bestimmen.

14. System nach Anspruch 1, wobei das computerimplementierte Verfahren ferner konfiguriert ist, um die Schmerzeinschätzung vor der Abgabe von dem Patienten, die Schmerzeinschätzung nach der Abgabe von dem Patienten, den Satz von Behandlungsdosisparametern, die der Schmerzeinschätzung vor der Abgabe und der Schmerzeinschätzung nach der Abgabe entsprechen, und jedwede Anpassungen zu empfangen, die von dem Patienten oder der Pflegeperson des Patienten an den Behandlungsdosisparametern während der Abgabe vorgenommen wurden.

## Revendications

1. Système pour traiter la douleur d'un patient en utilisant un blocage nerveux haute fréquence, le système comprenant :
un neuromodulateur implantable (271) comprenant :
une ou plusieurs électrodes configurées pour appliquer une modulation neuronale haute fréquence à un ou plusieurs nerfs ; et
un dispositif de commande (105, 131, 231) configuré pour appliquer une dose de traitement présentant un ensemble de paramètres de dose de traitement à partir des une ou plusieurs électrodes ; et
un sélecteur de dose (280) comprenant :
un ou plusieurs processeurs ;
une base de données de dosage spécifique à une patiente (409) de doses précédemment administrées et d'estimations correspondantes d'un changement des niveaux de douleur rapportés par le patient avant et après l'administration de chaque dose ; et
une mémoire couplée aux un ou plusieurs processeurs, la mémoire étant configurée pour stocker des instructions de programme informatique qui, lorsqu'elles sont exécutées par les un ou plusieurs processeurs, exécutent un procédé mis en oeuvre par ordinateur comprenant les étapes consistant à :
mettre à jour la base de données de dosage spécifique à un patient (409) avec une estimation de la douleur avant administration de la part du patient, une estimation de la douleur après administration de la part du patient, l'ensemble de paramètres de dose de traitement correspondant à l'estimation de la douleur avant administration et à l'estimation de la douleur après administration, et tout ajustement apporté à l'ensemble de paramètres de dose de traitement par le patient ou le soignant du patient pendant l'administration ; et
générer l'ensemble de paramètres de dose de traitement sur la base de la base de données de dosage spécifique à un patient (409).

2. Système selon la revendication 1, comprenant en outre une interface utilisateur configurée pour permettre l'entrée par le patient de l'estimation de la douleur avant administration et de l'estimation de la douleur après administration.

3. Système selon la revendication 2, dans lequel le système est configuré pour empêcher le dispositif de commande d'appliquer la dose de traitement jusqu'à ce que le patient ait entré l'estimation de la douleur avant administration dans l'interface utilisateur.

4. Système selon la revendication 2, dans lequel le système est configuré pour inviter automatiquement le patient à estimer la douleur après l'administration un temps prédéterminé après que la dose de traitement a été appliquée.

5. Système selon la revendication 1, dans lequel le dispositif de commande est configuré pour ajuster l'ensemble de paramètres de dose de traitement pendant l'administration sur la base de l'entrée du patient ou du soignant du patient.

6. Système selon la revendication 1, dans lequel le procédé mis en oeuvre par ordinateur comprend en outre une transmission de l'ensemble de paramètres de dose de traitement au dispositif de commande (105, 231) du neuromodulateur implantable (271).

7. Système selon la revendication 1, dans lequel la génération de l'ensemble de paramètres de dose de traitement sur la base de la base de données spécifique à un patient (241, 241', 411) comprend une génération d'un pic de tension, d'une fréquence et d'une durée de traitement.

8. Système selon la revendication 1, dans lequel la génération de l'ensemble de paramètres de dose de traitement sur la base de la base de données spécifique à un patient (241, 241', 411) comprend une identification d'un ensemble de paramètres de dose antérieurs à partir de la base de données de dosage spécifique à un patient (409) présentant une réduction de score élevée dans les niveaux de douleur rapportés par le patient avant et après administration en utilisant les paramètres de dose antérieurs ; et une modification des un ou plusieurs des paramètres de dose antérieurs par une quantité d'oscillation dans l'ensemble de paramètres de dose antérieurs pour former l'ensemble de paramètres de dose de traitement.

9. Système selon la revendication 8, dans lequel la quantité d'oscillation comprend un pourcentage de la valeur des un ou plusieurs paramètres de dose antérieurs.

10. Système selon la revendication 8, comprenant en outre un réglage de la quantité d'oscillation sur la base de l'identification d'une tendance dans les un ou plusieurs paramètres de dose à partir de la base de données de dosage spécifique à un patient (409).

11. Système selon la revendication 1, dans lequel le sélecteur de dose (280) fait partie de l'implant.

12. Système selon la revendication 1, dans lequel le sélecteur de dose fait partie d'un dispositif de commande de patient et/ou d'un serveur distant (261).

13. Système selon la revendication 1, dans lequel le procédé mis en oeuvre par ordinateur est en outre configuré pour déterminer des doses ultérieures en utilisant la base de données de dosage spécifique à un patient (409).

14. Système selon la revendication 1, dans lequel le procédé mis en oeuvre par ordinateur est en outre configuré pour recevoir l'estimation de la douleur avant administration de la part du patient, l'estimation de la douleur après administration de la part du patient, l'ensemble de paramètres de dose de traitement correspondant à l'estimation de la douleur avant administration et à l'estimation de la douleur après administration, et tout ajustement des paramètres de dose de traitement effectué par le patient ou le soignant du patient pendant l'administration.
